# FASCICULE DE BREVET EUROPEEN

(11) **EP 3 815 183 B1**
(45) Date de publication et mention de la délivrance du brevet: **07.06.2023**
(21) Numéro de dépôt: 19736628.9
(22) Date de dépôt: 27.06.2019
(51) Int. Cl.: H01Q 23/00, H01Q 1/38, H01Q 1/27, H01Q 1/22, H01Q 1/24, A61B 5/00, H05K 1/16, H05K 1/02, H05K 1/18

(54) **CIRCUIT IMPRIMÉ POUR LA COMMUNICATION DE DONNÉES**
GEDRUCKTE SCHALTUNG FÜR DATENKOMMUNIKATION
PRINTED CIRCUIT FOR DATA COMMUNICATION

(30) Priorité: 27.06.2018 FR 1855750
(43) Date de publication de la demande: 05.05.2021
(73) Titulaire: Centre national de la recherche scientifique, 75016 Paris (FR); UNIVERSITE DE MONTPELLIER, 34090 Montpellier (FR); Bonetag, 66000 Perpignan (FR)
(72) Inventeur: VENA, Arnaud, 34270 SAINT-MATHIEU-DE-TREVIERS (FR); SORLI, Brice, 34530 MONTAGNAC (FR); CAHUZAC, Stéphan, 34560 VILLEVEYRAC (FR); DUTRIEUX, Sylvain, 34270 Le Triadou (FR); PIGEON, Mélusine, T12 C2P2 Cork (IE)
(74) Mandataire: IPAZ
(86) Numéro de dépôt international: PCT/EP2019/067268
(87) Numéro de publication internationale: WO 2020/002564

(56) Documents cités:
- EP-A1- 2 023 275
- WO-A1-2004/042868
- WO-A1-2010/051217
- WO-A1-2017/082652
- WO-A1-2017/194096
- US-B1- 9 672 393
- ANTHONY NIKOLA LASKOVSKI ET AL: "Stacked Spirals for Biosensor Telemetry", IEEE SENSORS JOURNAL, IEEE SERVICE CENTER, NEW YORK, NY, US, vol. 11, no. 6, 1 juin 2011 (2011-06-01), pages 1484-1490, XP011354646, ISSN: 1530-437X, DOI: 10.1109/JSEN.2010.2091123

## Description

L'invention concerne un circuit imprimé pour la communication de données et une prothèse comprenant un tel circuit.

Le domaine de l'invention est le domaine des dispositifs de communications, et plus particulièrement, le domaine des dispositifs de communication prévus pour des implants médicaux, en particulier des implants utilisés dans des prothèses, de préférence les prothèses orthopédiques.

### ETAT DE LA TECHNIQUE

On connaît les prothèses comprenant un circuit imprimé, tel qu'une radio-étiquette qui peuvent être associés à des capteurs de grandeurs physiques comme la température, la pression, etc. Ces circuits imprimés permettent de transmettre entre autre les données mesurées par les capteurs en vue du suivi d'une caractéristique relative à la prothèse, comme la température ambiante, les efforts subis par la prothèse, etc.

De tels circuits imprimés comprennent au moins une antenne pour communiquer des données de façon non filaire et recevoir l'énergie pour alimenter le circuit imprimé. La performance de la réception d'énergie et de la communication de données de ces circuits dépendent des caractéristiques de l'antenne, telles que ces dimensions, sa géométrie, etc. En particulier, l'efficacité de l'antenne augmente avec ses dimensions. WO 2004/042868 A1, WO 2017/082652 A1, EP 2 023 275 A1 et US 9 672 393 B1 divulguent différentes structures d'antennes boucle sur des substrats diélectriques de circuits intégrés. ANTHONY NIKOLA LASKOVSKI ET AL : "Stacked Spirals for Biosensor Telemetry", IEEE SENSORS JOURNAL, IEEE SERVICE CENTER, NEW YORK, NY, US, vol. 11, no. 6, 1 juin 2011 (2011-06-01), pages 1484-1490, divulgue une bobine de transmission de puissance inductive pour des implants télémétriques basés sur des biocapteurs.

Ces circuits imprimés et leurs antennes restent encombrants pour une application dans les prothèses.

Un but de la présente invention est de proposer un circuit imprimé pour la communication de données moins encombrant.

### EXPOSE DE L'INVENTION

Au moins un des objectifs précités est atteint par un circuit imprimé tel que défini dans la revendication 1.

Avec le circuit imprimé selon l'invention, l'antenne est formée par des couches empilées suivant un plan perpendiculaire au support principal et le composant électronique est agencé à distance de ladite antenne. Le circuit imprimé se présente ainsi sous une forme tridimensionnelle. La surface sur laquelle s'étend le circuit imprimé est réduite. Le circuit imprimé selon l'invention est ainsi moins encombrant.

Au moins une extrémité de la piste conductrice formant l'antenne peuvent être reliées au composant électronique dans le plan principal.

De préférence, le support principal peut être une couche de substrat en matériau diélectrique. Le circuit imprimé peut comprendre au moins une autre couche de substrat en matériau diélectrique séparant deux couches empilées consécutives de l'antenne.

En particulier, les couches empilées de la piste conductrice peuvent être des couches conductrices, telles que des couches métalliques, déposées sur un matériau diélectrique.

Le composant électronique peut être une puce électronique configurée pour être alimentée par l'énergie reçue par l'antenne et transmettre des données, en particulier des données d'un capteur relié à la puce, vers un récepteur distant à travers ladite antenne.

Le composant électronique peut être de type passif.

Le composant électronique peut être un module I2C, SPI ou encore UART, pour des capteurs numériques et analogiques ou tout autre type de composant électronique.

L'antenne est du type antenne boucle. L'antenne est sensible à un champs magnétique parallèle au plan principal.

Préférentiellement, la distance entre le composant électronique et l'antenne peut être inférieure à 50 mm.

Dans un mode de réalisation du circuit imprimé selon l'invention, au moins une face du support principal peut comprendre une couche de l'antenne.

En particulier, chaque face du support principal peut comprendre une couche de l'antenne.

Les couches de l'antenne peuvent être déposées sur une ou les deux faces du support principal, par toute technique de dépôt de couche conductrice telle que la lithographie, la gravure et/ou la pulvérisation, etc.

Dans un mode de réalisation du circuit imprimé selon l'invention, l'antenne peut comprendre plusieurs couches et toutes les couches de l'antenne peuvent être empilées d'un même côté par rapport à une face du support principal, en particulier une face opposée à celle sur laquelle est disposée le composant électronique.

Alternativement, toutes les couches de l'antenne peuvent être empilées d'un même côté par rapport à une face du support principal sur laquelle est disposée le composant électronique.

Les couches de l'antenne peuvent être empilées dans la direction du composant électronique ou dans une direction opposée à celle-là.

Dans un mode de réalisation préféré du circuit imprimé selon l'invention, toutes les couches de l'antenne peuvent être empilées vers le composant électronique à partir de la face du support principal opposée à celle comportant ledit composant.
Préférentiellement, l'antenne peut être répartie sur quatre couches empilées selon la direction perpendiculaire au plan principal.

Dans une version de réalisation du circuit imprimé selon l'invention, l'antenne peut être une antenne boucle formée par deux enroulements, un enroulement extérieur et un enroulement intérieur.

Ainsi, l'antenne est moins sensible aux perturbations extérieures telles que la proximité de métaux. Le circuit imprimé selon l'invention est plus robuste.

L'enroulement extérieur peut être formé par les couches empilées extérieures de l'antenne et l'enroulement intérieur peut être formé par les couches empilées intérieures de l'antenne.

Les enroulements intérieur et extérieur peuvent être imbriqués, en particulier concentriques.

L'antenne est sensible à un champ magnétique parallèle au plan principal.

Avantageusement, le circuit imprimé selon l'invention peut comprendre au moins un support, dit secondaire, comprenant au moins une couche de l'antenne et agencé sur ledit support principal.

Le support secondaire peut être agencé sur la face du support principale comportant le composant électronique.

Suivant un mode de réalisation préféré du circuit imprimé selon l'invention, au moins un support secondaire peut être amovible.

En particulier, le support secondaire peut être amovible avec ou sans outils.

Le deuxième support peut être relié au support principal par tout moyen d'assemblage tel que par brasure, vissage, clips, collage, collage par colle conductrice, par exemple la prepreg ROGERS 4550, etc.

Préférentiellement, le circuit imprimé selon l'invention peut comprendre deux supports secondaires et au moins une couche de l'antenne peut être agencée entre lesdits supports secondaires.

L'au moins une couche de l'antenne peut être agencée sur une face du support principale, en regard du support secondaire, ou sur une face du support secondaire en regard du support principal.

Dans une version de réalisation du circuit imprimé selon l'invention, au moins une couche de l'antenne peut être agencée sur une face d'un support secondaire opposée à l'autre support secondaire.

En particulier, la largeur d'au moins un support secondaire peut être comprise entre la largeur de la piste conductrice et la largeur du support principal moins la largeur du composant électronique.

Dans un mode de réalisation particulier du circuit imprimé selon l'invention, la piste conductrice peut comprendre au moins un moyen de liaison, dit principal, au niveau du support principal et au moins un moyen de liaison, dit secondaire, au niveau d'un support secondaire, lesdits moyens de liaison étant prévus pour relier la piste conductrice entre le support principal et ledit support secondaire.

Les moyens de liaison principal et secondaire peuvent être tout moyen de connexion électrique par exemple un premier dépôt métallique sur le support principal coopérant avec un deuxième dépôt métallique pour assurer la liaison électrique de la piste conductrice.

Avantageusement, la piste conductrice peut comprendre au moins un via reliant deux couches empilées.

Au moins un via peut relier deux couches empilées adjacentes ou non.

En particulier, la piste peut comprendre deux vias traversant le support principal et les supports secondaires et reliant les couches empilées extérieures de l'antenne pour former l'enroulement extérieur. La piste peut aussi comprendre deux vias traversant un support secondaire et reliant les couches empilées intérieures de l'antenne pour former l'enroulement intérieur.

En outre, l'enroulement extérieur et l'enroulement intérieur peuvent être reliés entre eux par au moins un via traversant le support principal ou un support secondaire.

La largeur de la piste conductrice peut être inférieure 2 mm, en particulier comprise entre 0.2 mm et 0.5 mm.

Dans une version avantageuse de réalisation du circuit imprimé selon l'invention, une couche empilée externe de l'antenne peut être à la même hauteur que la limite supérieure du composant électronique.

Avantageusement, l'antenne peut communiquer des données depuis et/ou vers le composant électronique vers et/ou depuis un dispositif distant. L'antenne peut en outre alimenter le composant électronique.

Dans une version de réalisation du circuit imprimé selon l'invention, le composant électronique peut être relié à un capteur configuré pour mesurer au moins une donnée relative à l'environnement du circuit imprimé.

Le capteur peut être un capteur d'effort, de pression, de position, de température, bactériologique, etc. L'antenne peut être configurée pour alimenter et/ou transmettre les données dudit capteur.

En particulier, le circuit imprimé peut comprendre des connecteurs prévus pour relier le capteur au composant électronique. Ces connecteurs peuvent être agencés sur une face du support principal, opposée à la face comprenant le composant électronique.

Avantageusement, le circuit imprimé peut être une radio-étiquette tridimensionnelle équipant un implant orthopédique.

Dans un mode de réalisation, le circuit imprimé selon l'invention peut comprendre une pluralité d'antennes telles que décrites ci-dessus. Au moins deux antennes peuvent être sensibles à des champs magnétiques dans des directions orthogonales entre elles et parallèles au plan principal du circuit imprimé.

Cette configuration permet d'augmenter le rayonnement des antennes et donc d'améliorer la communication de données du circuit imprimé et l'alimentation du circuit imprimé selon l'invention.

Par exemple, les antennes peuvent être agencées selon un système MIMO (« multiple input multiple output » en anglais) pour obtenir une meilleure couverture en rayonnement.

En outre, le circuit imprimé peut comprendre quatre antennes agencées en quadrature, ce qui permet de réaliser une polarisation circulaire et obtenir une meilleure réception et aussi d'optimiser la communication (émission et réception).

Dans un mode de réalisation, le circuit imprimé et son antenne selon l'invention peut comprendre au moins deux composant électroniques.

Ainsi, le circuit imprimé et son antenne selon l'invention peut comprendre plusieurs couches de l'antenne agencées pour intégrer au moins deux composants électroniques, par exemple sur un même niveau dans le circuit imprimé, ou de préférence, suivant plusieurs niveaux dans le circuit imprimé.

Le circuit imprimé et son antenne selon l'invention peut comprendre plusieurs couches de l'antenne agencées pour intégrer au moins deux composants électroniques qui sont positionnés sur un même niveau dans le circuit imprimé.

Alternativement et/ou en combinaison, le circuit imprimé et son antenne selon l'invention peut comprendre plusieurs couches de l'antenne agencées pour intégrer au moins deux composants électroniques qui sont positionnés sur deux niveaux différents dans le circuit imprimé.

Selon un autre aspect de l'invention, il est proposé une prothèse comprenant un circuit imprimé selon l'invention.

La prothèse peut être tout type de prothèses telle qu'une prothèse orthopédique, articulaire, etc.

Dans une version de réalisation avantageuse, la prothèse selon l'invention peut comprendre un capteur configuré pour mesurer au moins une donnée relative à ladite prothèse.

En particulier, le circuit imprimé peut être agencé dans un boîtier étanche et le capteur peut être agencé à l'extérieur dudit boîtier en contact d'un tissu organique prévu pour mesurer des données relatives audit tissu.

### DESCRIPTION DES FIGURES ET MODES DE REALISATION

D'autres avantages et caractéristiques apparaîtront à l'examen de la description détaillée d'exemples de réalisation nullement limitatifs, et des dessins annexés sur lesquels :
- la FIGURE 1 est une représentation schématique d'un premier exemple du circuit imprimé selon l'invention ;
- les FIGURES 2a et 2b sont des représentations schématiques d'un deuxième exemple du circuit imprimé selon l'invention et de son antenne, respectivement ;
- les FIGURES 3a et 3b sont des représentations schématiques d'un troisième exemple du circuit imprimé selon l'invention et de son antenne, respectivement ,
- la FIGURE 4 est une représentation schématique d'un quatrième exemple du circuit imprimé selon l'invention ,
- la FIGURE 5 est une représentation schématique d'un cinquième exemple du circuit imprimé selon l'invention,
- les FIGURES 6a, 6b et 6c sont des représentations schématiques d'un exemple d'une antenne pouvant équiper un circuit imprimé selon l'invention (mode de réalisation non couvert par les revendications),
- les FIGURES 7a, 7b et 7c sont des représentations schématiques d'un autre exemple d'une antenne pouvant équiper un circuit imprimé selon l'invention (mode de réalisation non couvert par les revendications),
- les FIGURES 8a, 8b et 8c sont des représentations schématiques d'un autre exemple d'un circuit imprimé selon l'invention et de son antenne, respectivement,
- les FIGURES 9a, 9b et 9c sont des représentations schématiques d'un autre exemple du circuit imprimé et de son antenne de la FIGURE 8 insérés dans une cavité métallique,
- la FIGURE 10 est une représentation schématique d'un autre exemple de cavité métallique pour l'antenne et le circuit imprimé de la FIGURE 8,
- la FIGURE 11 est une représentation schématique d'un autre exemple de cavité métallique pour l'antenne et le circuit imprimé de la FIGURE 8,
- la FIGURE 12 est une représentation schématique d'un autre exemple de cavité métallique pour l'antenne et le circuit imprimé de la FIGURE 8,
- les FIGURES 13a, 13b et 13c sont des représentations schématiques d'un autre exemple d'un circuit imprimé selon l'invention et de son antenne, respectivement, et
- les FIGURES 14a, 14b, 14c et 14d sont des représentations schématiques d'un autre exemple d'un circuit imprimé selon l'invention et de son antenne, respectivement.

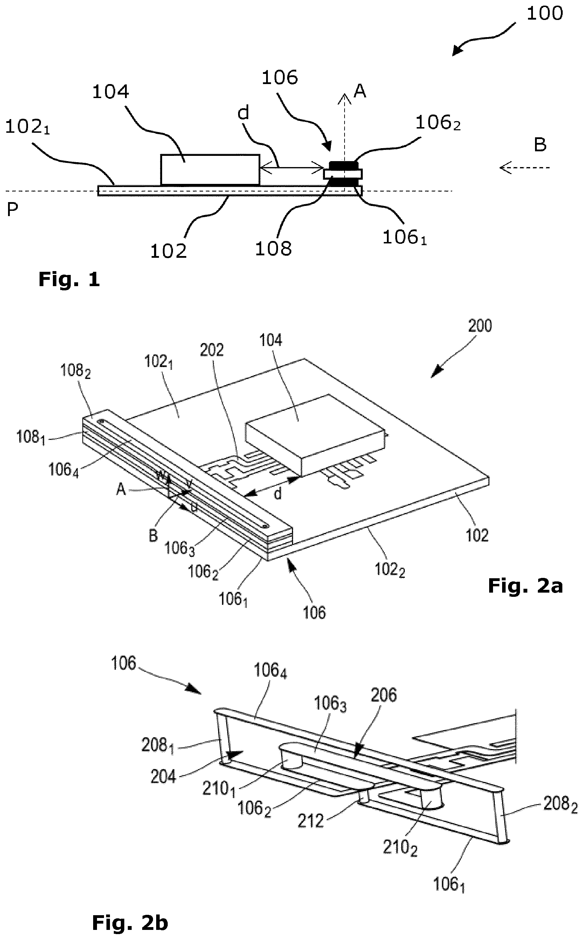

La FIGURE 1 est une représentation schématique d'un premier exemple du circuit imprimé selon l'invention.

Le circuit imprimé 100 comprend un support principal 102 formant un plan P, dit principal. Le support principal 102 est une couche de substrat du circuit imprimé 100 réalisée dans un matériau diélectrique.

Le circuit imprimé 100 comprend un composant électronique 104, par exemple une puce électronique, agencé sur une face 102₁ du support principal 102.

Le circuit imprimé 100 comprend en outre une antenne 106, en particulier une antenne boucle, formée par une piste conductrice répartie sur deux couches empilées 106₁ et 106₂ selon une direction A orthogonale au plan P. L'antenne 106 produit ainsi un courant électrique lorsqu'elle est soumise à un champ magnétique B parallèle au plan P et inversement.

Le composant électronique 104 et l'antenne 106 sont agencés à une distance d, non nulle, l'un de l'autre dans le plan P. La distance d est inférieure à 100mm.

La couche empilée 106₁ est disposée sur la face 102₁ du support principal 102, celle qui comprend le composant électronique 104.

Le circuit imprimé 100 comprend un deuxième support 108, dit secondaire, agencé sur le support principal 102 et comprenant la couche empilée 106₂. Le support secondaire 108 est aussi une couche de substrat du circuit imprimé 100 réalisée dans un matériau diélectrique.

Par exemple, les couches empilées 106 sont réalisées par des procédés de gravure.

Le support principal 102 et le support secondaire 108 sont collées entre eux, par exemple avec une brasure à l'étain.

L'antenne 106 est reliée au composant électronique 104 et est configurée pour l'alimenter et/ou communiquer des données entre le composant électronique 104 et un lecteur distant.

Le circuit imprimé 100 peut être intégré dans un implant en particulier un implant pour une prothèse orthopédique.

Les FIGURES 2a et 2b sont des représentations schématiques d'un deuxième exemple du circuit imprimé selon l'invention et de son antenne, respectivement.

Le circuit imprimé 200 des FIGURES 2a et 2b comprend les mêmes éléments que le circuit imprimé 100 de la FIGURE 1. A la différence, l'antenne 106 du circuit imprimé 200 est formée par quatre couches empilées 106₁ à 106₄.

Le support principal 102 comprend, sur chacune de ses faces 102₁ et 102₂, une couche empilée 106₂, respectivement 106₁.

Les couches 106₁-106₄ de l'antenne 106 sont empilées dans une seule direction A en partant de la face 102₂ du circuit imprimé 200.

Le circuit imprimé 200 comprend aussi deux supports secondaires 108₁ et 108₂, comprenant chacun une couche empilée 106₃ et 106₄ respectivement.

La couche empilée 106₄ est agencée sur une face du support secondaire 108₂ opposée au support secondaire 108₁ de sorte à former une couche extérieure de l'antenne 106.

L'antenne 106 est une antenne boucle comprenant un premier enroulement extérieur 204 formé par les couches 106₁ et 106₄ et un deuxième enroulement intérieur 206 formé par les couches 106₂ et 106₃.

L'antenne 106 comprend deux vias 208₁ et 208₂ traversant le support principal 102 et les supports secondaires 108₁ et 108₂ pour former l'enroulement extérieur 204. De même, l'antenne 106 comprend deux vias 210₁ et 210₂ traversant le support secondaire 108₁ pour former l'enroulement intérieur 206.

L'enroulement intérieur 206 et l'enroulement extérieur 204 sont reliés entre eux par un via 212 traversant le support principal 102.

Les vias 208, 210 et 212 se présentent sous la forme de trous métalliques réalisés dans le(s) support(s) qu'ils traversent.

La couche 106₄ extérieure de l'antenne 106 est agencée à la même hauteur que la limite supérieure du composant électronique 104. Le circuit imprimé 200 est ainsi plus compact.

Le circuit imprimé 200 comprend en outre, sur sa face 102₁, des connecteurs 202, sous la forme d'une piste électrique, pour relier l'antenne 106 au composant électronique 104.

En outre, l'antenne 106 est formée par un empilement de couches suivant une direction d'empilement perpendiculaire au plan P du circuit imprimé 200. Par conséquent, l'antenne boucle 106 se trouve dans un plan perpendiculaire au plan du circuit imprimé 200. Ainsi, le fonctionnement de l'antenne 106 ne peut être perturbé que par une couche métallique perpendiculaire au plan du circuit imprimé 200, et parallèle au plan formé par l'antenne boucle 106, et qui se trouve devant l'antenne 106 dans le sens de propagation du champ magnétique B. Dans tous les autres cas, la présence d'une couche métallique ne vient pas perturber le fonctionnement de l'antenne.

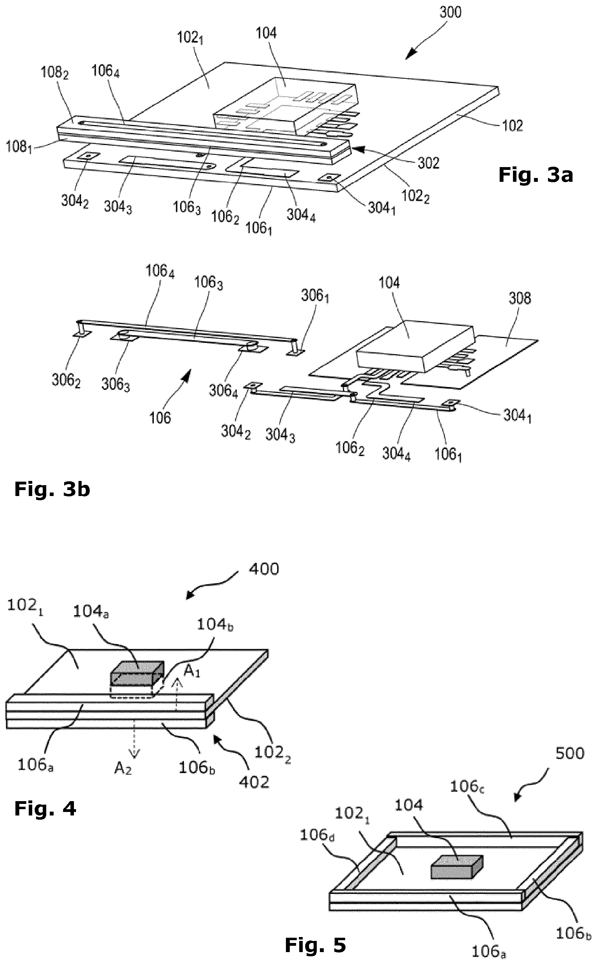

Les FIGURES 3a et 3b sont des représentations schématiques d'un troisième exemple du circuit imprimé selon l'invention et de son antenne, respectivement.

Le circuit imprimé 300 comprend les mêmes éléments que le circuit imprimé 200 des FIGURES 2a et 2b. A la différence, les supports secondaires 108₁ et 108₂ forment un ensemble 302 amovible.

Le circuit imprimé 300 comprend des moyens de liaison 304₁-304₄, dit principaux, au niveau du support principal 102 coopérant avec des moyens de liaison 306₁-306₄, dit secondaires, au niveau de l'ensemble 302 pour former l'antenne 106.

Les moyens de liaison principaux 304 sont agencées sur la face 102₁ et les moyens de liaison secondaires 306 sont agencées sur une face du support secondaire 108₁ en regard de la face 102₁.

Les moyens de liaison principaux 304 et secondaires 306 se présentent sous la forme d'un dépôt métallique configuré pour assurer la connexion électrique de l'antenne 106.

Les moyens de liaison principaux 304₁ et 304₂ coopèrent avec les moyens de liaison 306₁ et 306₂ pour former l'enroulement extérieur 204, tandis que les moyens de liaison principaux 304₃ et 304₄ coopèrent avec les moyens de liaison 306₃ et 306₄ pour former l'enroulement intérieur 206.

Le support principal 102 et les supports secondaires 108 peuvent être fabriqués séparément et assemblés par la suite. La réalisation du circuit imprimé 300 est ainsi moins coûteuse.

Le circuit imprimé 300 comprend aussi des pistes de connexion 308 prévues pour relier le composant électronique 104 à un capteur, par exemple un capteur de pression.

De plus, le circuit imprimé 300 peut être intégré dans un implant en particulier un implant pour une prothèse orthopédique. Le capteur peut être configuré pour mesurer des données relatives audit implant, par exemple sa température, les efforts qu'il subit, etc. l'antenne 106 transmet ses données à un lecteur distant, par exemple pour assurer un suivi dudit implant en vue son remplacement.

La FIGURE 4 est une représentation schématique d'un quatrième exemple du circuit imprimé selon l'invention.

Le circuit imprimé 400 comprend les mêmes éléments que le circuit imprimé 300 des FIGURES 3a et 3b. A la différence, le circuit imprimé 400 comprend deux antennes 106ₐ et 106_{b} et deux composants électroniques 104ₐ et 104_{b}.

Les composants électroniques 104ₐ et 104_{b} sont agencés sur deux faces opposées du support principal 102, respectivement 102₁ et 102₂. Les composants électroniques 104ₐ et 104_{b} peuvent être agencés sur une même face du support principal 102. Les composants électroniques 104ₐ et 104_{b} peuvent être identiques ou avec des fonctionnalités différentes.

L'antenne 106ₐ est agencée sur la face 102₁ et les couches empilées de l'antenne 106ₐ sont empilées en partant de la face 102₁ dans une direction A₁ perpendiculaire au plan du circuit imprimé 400 en direction du composant électronique 104ₐ.

L'antenne 106_{b} est agencée sur la face 102₁ et les couches empilées de l'antenne 106_{b} sont empilées en partant de la face 102₂ dans une direction A₂ opposée à la direction A₁.

Les antennes 106ₐ et 106_{b} sont agencées au niveau d'un même bord 402 du circuit imprimé 400. Les 106ₐ et 106_{b} peuvent être agencées de côtés du circuit imprimé 400, par exemple sur deux bords adjacents ou opposés entre eux.

Chaque antenne 106ₐ et 106_{b} est reliée aux composants électroniques 104ₐ et 104b.

Alternativement, ou en plus, chaque antenne 106 peut être reliée à un unique composant électronique 104.

Le circuit imprimé 400 est ainsi moins encombrant pour un nombre de composants électroniques plus important comparé aux circuits imprimés de l'état de la technique.

La FIGURE 5 est une représentation schématique d'un cinquième exemple du circuit imprimé selon l'invention.

Le circuit imprimé 500 comprend les mêmes éléments que le circuit imprimé 300 des FIGURES 3a et 3b. A la différence, le circuit imprimé 400 comprend quatre antennes 106ₐ, 106_{b}, 106_{c} et 106_{d}.

Les antennes 106 sont agencées sur une même face 102₁ du circuit imprimé 500 et sont distribuées sur chaque bord du circuit imprimé 500.

Les antennes 106 sont agencées en quadrature. Cette configuration permet ainsi de réaliser une polarisation circulaire et donc d'obtenir une meilleure communication pour un circuit imprimé 500 plus compact.

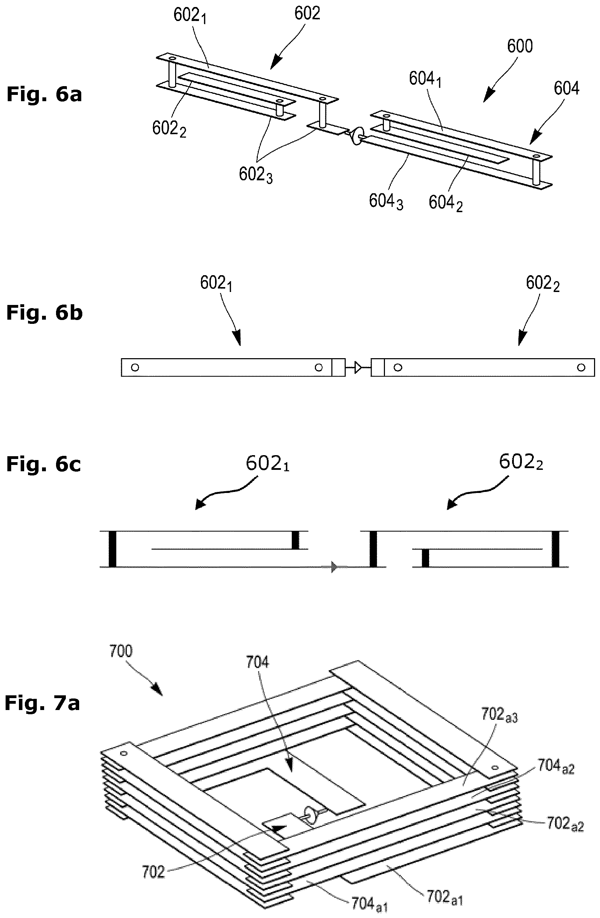

Les FIGURES 6a, 6b et 6c sont des représentations schématiques d'un exemple d'une antenne pouvant équipé un circuit imprimé (mode de réalisation non couvert par les revendications).

Les FIGURES 6a, 6b et 6c sont, respectivement, des vues en perspectives, de haut et de face de l'antenne 600.

L'antenne 600 est de type antenne dipôle et peut remplacer l'antenne 106 des circuits imprimés 100, 200, 300, 400 ou 500.

L'antenne 600 comprend deux pôles 602 et 604 distincts. Chaque pôle 602 et 604 est formé par une piste électrique répartie sur trois couches 602₁, 602₂ et 602₃, respectivement 604₁, 604₂ et 604₃. Les couches 602₁-602₃ et 604₁-604₃ peuvent être déposées sur une face d'une couche de substrat du circuit imprimé.

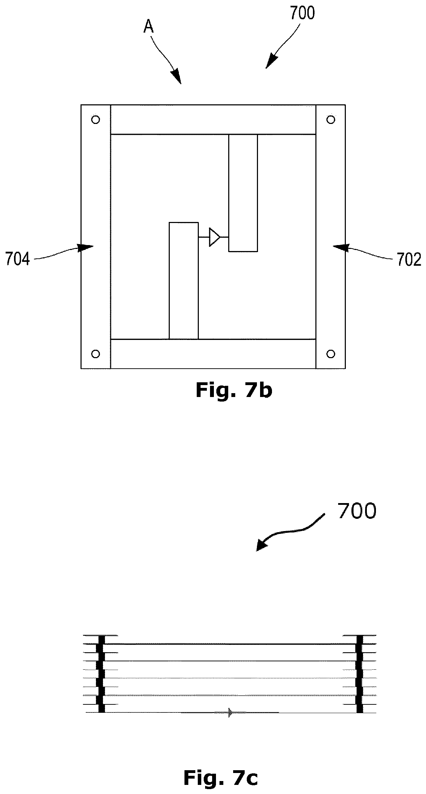

Les FIGURES 7a, 7b et 7c sont des représentations schématiques d'un autre exemple d'une antenne pouvant équipé un circuit imprimé (mode de réalisation non couvert par les revendications).

Les FIGURES 7a, 7b et 7c sont, respectivement, des vues en perspectives, de haut et de face de l'antenne 700.

L'antenne 700 est de type antenne hélicoïdale et peut remplacer l'antenne 106 des circuits imprimés 100, 200, 300, 400 ou 500. De préférence, le circuit imprimé présente une forme rectangulaire ou carré.

En particulier, l'antenne 700 peut être agencée de sorte à occuper les bords du circuit imprimé.

L'antenne 700 comprend deux antennes distinctes 702 et 704 formant chacune une hélice autour du circuit imprimé.

Chaque antenne 702 et 704 est formée par une piste électrique répartie sur plusieurs couches autour du circuit imprimé de sorte que deux couches successives sont agencées sur deux bords adjacents du circuit imprimé et sont reliées entre elles au niveau de la jonction de ces deux bords. Les antennes 702 et 704 sont réparties autour de la périphérie du circuit imprimé selon deux directions de rotation opposées et sont imbriquées entre elles de façon semblable à un escalier à double révolution.

Ainsi, pour chaque bord du circuit imprimé, par exemple le bord A, l'antenne 702 comprend des couches 702ₐ₁, 702ₐ₂, 702ₐ₃ agencées en alternance avec des couches 704ₐ₁, 704ₐ₂ de l'antenne 704.

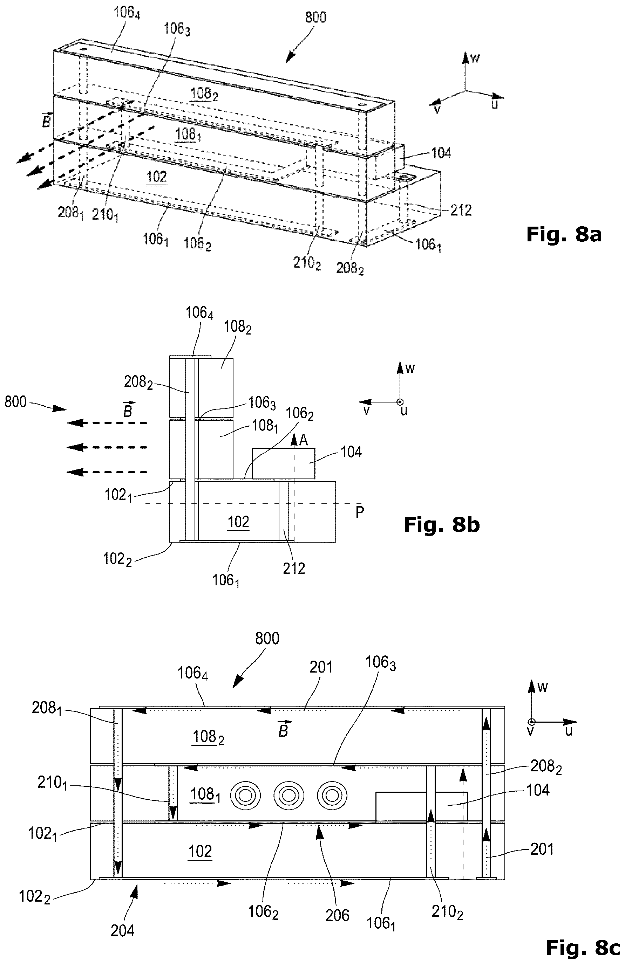

Les FIGURES 8a, 8b et 8c sont des représentations schématiques d'un huitième exemple du circuit imprimé selon l'invention et de son antenne, respectivement.

Le circuit imprimé 800 des FIGUES 8a, 8b et 8c sont une même variante de l'enroulement de l'antenne 106 présenté en FIGURES 2a et 2b. Ainsi, seules les différences avec les FIGURES 2a et 2b seront décrites.

La FIGURE 8a est notamment une vue de profil, appelée face avant, du circuit imprimé 800, ladite FIGURE 8a étant illustrée avec un substrat transparent de manière à distinguer les différents composants du circuit 800.

La FIGURE 8b est notamment une vue, dite face gauche du circuit imprimé 800, dans le plan (w, v), ayant un substrat transparent.

La FIGURE 8c est notamment une vue, dite face avant du circuit imprimé 800, dans le plan (w, u), ayant un substrat transparent.

L'antenne 106 du circuit imprimé 800 est formée par quatre couches empilées 106₁ à 106₄.

Le support principal 102 comprend, sur chacune de ses faces 102₁ et 102₂, une couche empilée 106₂, respectivement 106₁.

Les couches 106₁-106₄ de l'antenne 106 sont empilées dans une seule direction A en partant de la face 102₂ du circuit imprimé 800.

Le circuit imprimé 800 comprend aussi deux supports secondaires 108₁ et 108₂, comprenant chacun une couche empilée 106₃ et 106₄ respectivement.

La couche empilée 106₄ est agencée sur une face du support secondaire 108₂ opposée au support secondaire 108₁ de sorte à former une couche extérieure de l'antenne 106.

Sur les FIGURES 8a, 8b, 8c, l'antenne 106 comprend deux vias 208₁ et 208₂ traversant le support principal 102 et les supports secondaires 108₁ et 108₂ pour former un enroulement extérieur 204. De même, l'antenne 106 comprend deux vias 210₁ et 210₂.

Le via 210₂ traverse le support principal 102 et le support secondaire 108₁. Le via 210₁ traverse seulement le support secondaire 108₁. Les vias 210₁ et 210₂ forment un enroulement intérieur 206.

L'enroulement intérieur 206 et l'enroulement extérieur 204 sont reliés entre eux par le via 210₂ qui traverse le support principal 102. Le via 210₂ permet ainsi de connecter la couche 106₁ de l'enroulement extérieur 204 à la couche 106₃ de l'enroulement intérieur 206.

Le via 212 traverse seulement le support principal 102 et relie la couche 106₁ à la couche 106₂.

Comme pour les FIGURES 2a et 2b, les vias 208, 210 et 212 se présentent sous la forme de trous métalliques réalisés dans le(s) support(s) qu'ils traversent.

Toutefois, à la différence des FIGURES 2a et 2b, au niveau des FIGURES 8a, 8b et 8c, la couche 106₄ extérieure de l'antenne 106 n'est pas agencée à la même hauteur que la limite supérieure du composant électronique 104. En effet, en FIGURES 8a, 8b et 8c, le composant électronique 104, positionné sur le support principal 102, est en dessous de la limite supérieure du support secondaire 108₁.

Le circuit imprimé 800 comprend aussi, sur sa face 102₁, des connecteurs (non illustrés), sous la forme d'une piste électrique, pour relier l'antenne 106 au composant électronique 104.

Sur les FIGURES 2a à 2c et les FIGURES 8a à 8c, l'enroulement de l'antenne 106, par l'agencement de ses couches 106₁, 106₂, 106₃, 106₄, permet un transfert d'énergie optimal dans le plan parallèle au circuit imprimé 800. En effet, comme représenté en FIGURE 2c et FIGURE 8c, l'enroulement des couches 106₁, 106₂, 106₃, 106₄ par les vias 208, 210 crée un enroulement de l'antenne 106 perpendiculaire au plan principal P. Ainsi, cela permet au circuit imprimé 800 de privilégier un rayonnement suivant une direction dans le plan du circuit imprimé 800. Le courant I se propage selon une boucle dans les couches du circuit imprimé 800 en suivant une direction 201. Il produit ainsi un champ magnétique B, en champ proche, parallèle au plan P comme le montre les FIGURES 1, 2 et 8. L'antenne 106 rayonne en champ lointain selon une direction similaire au champ magnétique B, c'est-à-dire parallèle au plan P.

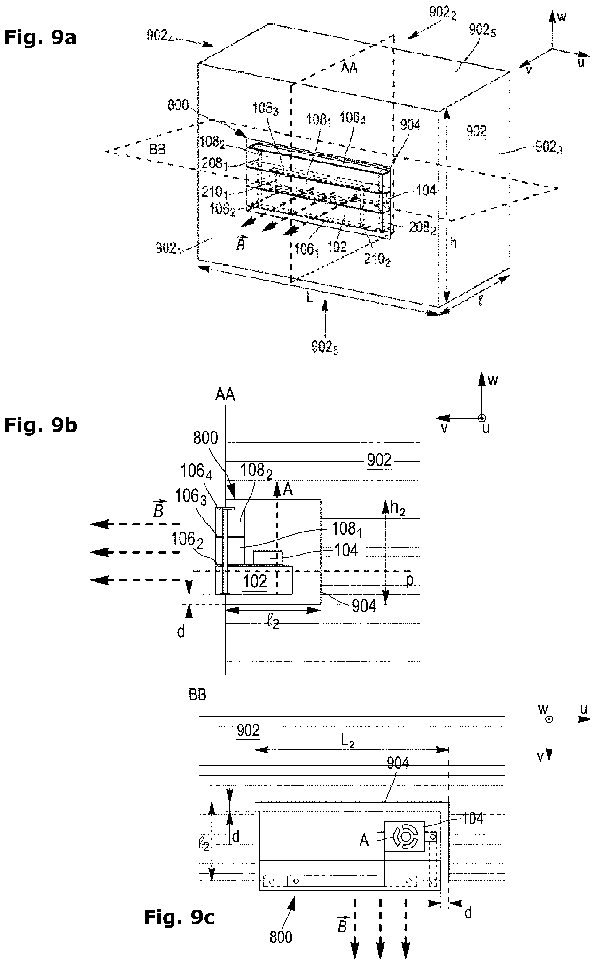

Les FIGURES 9a, 9b et 9c sont des représentations schématiques du circuit imprimé 800 selon l'invention et son antenne 106 dans une cavité métallique 902. Ainsi, seules les différences avec les FIGURES 8a, 8b et 8c seront décrites.

La FIGURE 9a est notamment une vue de profil, appelée face avant du circuit imprimé 800, ladite FIGURE 9a étant illustrée avec un substrat transparent de manière à distinguer les différents composants du circuit 800.

La FIGURE 9b est notamment une vue selon la coupe AA du circuit imprimé 800 de la FIGURE 9a et la FIGURE 9c est une vue selon la coupe BB du circuit imprimé 800 de la FIGURE 9.

La cavité métallique 902 est un pavé rectangulaire, composé de 6 faces :
- une face 902₁, appelée face avant du pavé rectangulaire,
- une face 902₂, appelée face arrière du pavé rectangulaire,
- une face 902s, appelée face droite du pavé rectangulaire,
- une face 902₄, appelée face gauche du pavé rectangulaire,
- une face 902s, appelée face supérieure du pavé rectangulaire, et
- une face 902₆, appelée inférieure avant du pavé rectangulaire.

La cavité métallique 902 comprend une longueur L de 40 mm, 20 mm de largeur l et 30 mm de hauteur h.

La cavité métallique 902 est percée par un trou 904 de forme rectangulaire sur la face avant 902₁.

Les dimensions du trou 904 de la cavité métallique sont une longueur L₂ de 24 mm, une largeur l₂ de 8 mm et une hauteur h₂ de 11 mm.

Le trou 904 est agencé de manière à recevoir le circuit imprimé 800 et son antenne 106 à l'intérieur de la cavité métallique 902. Ainsi en FIGURES 9a, 9b et 9c, seules cinq faces sur six du circuit imprimé 800 et de son antenne 106 sont recouvertes par une face métallique de la cavité métallique 902. La face non recouverte par une partie métallique est la face avant du circuit-imprimé 800 et de son antenne 106, c'est à dire la face comprenant en son extrémité l'antenne 106 du circuit imprimé 800 et donnant sur la face 902₁ de la cavité métallique 902.

De manière similaire aux FIGURES 8a à 8c, en FIGURES 9a, 9b et 9c, l'enroulement des couches 106₁, 106₂, 106₃, 106₄ par les vias 208, 210 crée un enroulement de l'antenne 106 perpendiculaire au plan principal P. Ainsi, cela permet au circuit imprimé 800 de privilégier un rayonnement suivant une direction dans le plan du circuit imprimé 800. En effet, sur les FIGURES 9a, 9b et 9c, le champ magnétique B, émis par l'antenne suite à une propagation du courant I dans les couches du circuit imprimé 800, est parallèle au plan P. Ainsi, la présence de métal sur cinq faces sur six du circuit imprimé 800 et de son antenne 106 n'altère pas les performances de rayonnement de l'antenne 106 tant qu'aucune plaque métallique de la cavité métallique 902 ne recouvre le plan formé par l'empilement des couches 106₁ à 106₄ de l'antenne 106 dans la direction A, c'est-à-dire, la face à l'extrémité du circuit imprimé 800 crée par l'antenne 106 et donnant sur l'évidement de_la face 902₁ de la cavité métallique 902.

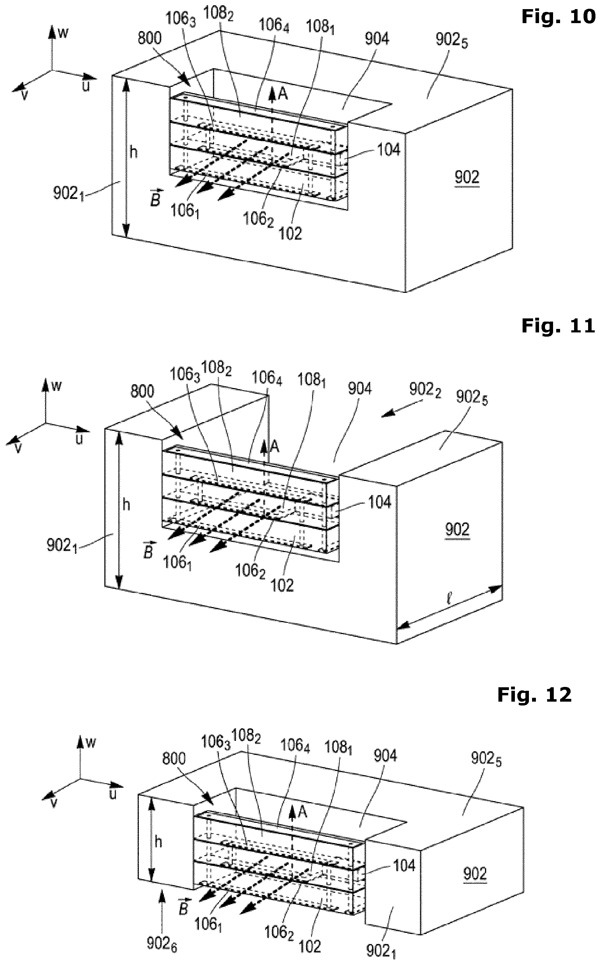

La FIGURE 10 est une représentation schématique d'un second exemple de circuit imprimé 800 et de son antenne 106 dans une cavité métallique 902. Ainsi, seules les différences avec les FIGURES 8a, 8b et 8c et les FIGURES 9a, 9b et 9c seront décrites.

La cavité métallique 902 est percée en son centre par un trou de forme rectangulaire et dans lequel le circuit imprimé 800 et son antenne 106 sont agencés.

A la différence des FIGURES 9a, 9b et 9c, seules quatre faces sur six du circuit imprimé 800 et de son antenne 106 sont recouvertes par une partie métallique de la cavité métallique 902. Ainsi la hauteur h de la cavité métallique 902 est diminuée d'un peu moins d'un tiers de la hauteur h de la cavité métallique 902 illustrée aux FIGURES 9a, 9b et 9c.

Les faces non recouvertes par une partie métallique sont :
- la face avant du circuit imprimé 800 et de son antenne 106, c'est à dire la face comprenant en son extrémité l'antenne 106 du circuit imprimé 800 et donnant sur la face 902₁ de la cavité métallique 902, et
- la face supérieure du circuit imprimé 800 et de son antenne 106, c'est à dire la face du circuit imprimé 800 donnant sur la face 902₅ de la cavité métallique 902.

Le champ magnétique B est parallèle au plan P. La présence de métal sur quatre faces sur six du circuit imprimé 800 n'altère pas les performances de rayonnement de l'antenne 106 tant qu'aucune plaque métallique de la cavité métallique 902 ne recouvre le plan formé par l'empilement des couches 106₁ à 106₄ de l'antenne 106 dans la direction A, c'est-à-dire la face à l'extrémité du circuit imprimé 800 crée par l'antenne 106 et donnant sur l'évidement de la face 902₁ de la cavité métallique 902.

La FIGURE 11 est une représentation schématique d'un troisième exemple de circuit imprimé 800 et de son antenne 106 dans une cavité métallique 902. Ainsi, seules les différences avec les FIGURES 8a, 8b et 8c et les FIGURES 9a, 9b et 9c seront décrites.

La cavité métallique 902 est percée en son centre par un trou 904 de forme rectangulaire et perçant toute la largeur l de la cavité métallique 902. Ainsi les largeurs l et l₂ sont identiques. Le circuit imprimé 800 et son antenne 106 sont agencés dans le trou 904 de la cavité métallique 902. La hauteur h de la cavité métallique 902 est similaire à celle de la FIGURE 10.

A la différence des FIGURES 9a, 9b et 9c et 10, seules trois faces sur six du circuit imprimé 800 et de son antenne 106 sont recouvertes par une partie métallique de la cavité métallique 902.

Les faces non recouvertes par une partie métallique sont :
- la face avant du circuit-imprimé 800 et de son antenne 106, c'est à dire la face comprenant en son extrémité l'antenne 106 du cuit-imprimé 800 et donnant sur la face 902₁ de la cavité métallique 902,
- la face supérieure du circuit-imprimé 800 et de son antenne 106, c'est à dire la face du circuit imprimé 800 donnant sur la face 902₅ de la cavité métallique 902, et,
- la face arrière du circuit-imprimé 800 et de son antenne 106, c'est à dire la face du circuit imprimé 800 donnant sur la face 902₂ de la cavité métallique 902.

Le champ magnétique B est parallèle au plan P. La présence de métal sur trois faces sur six du circuit imprimé 800 n'altère pas les performances de rayonnement de l'antenne 106 tant qu'aucune plaque métallique de la cavité métallique 902 ne recouvre le plan formé par l'empilement des couches 106₁ à 106₄ de l'antenne 106 dans la direction A, c'est-à-dire la face à l'extrémité du circuit imprimé 800 crée par l'antenne 106 et donnant sur l'évidement de la face 902₁ de la cavité métallique 902.

La FIGURE 12 est une représentation schématique d'un quatrième exemple de circuit imprimé 800 et de son antenne dans une cavité métallique 902. Ainsi, seules les différences avec les FIGURES 8a, 8b et 8c et les FIGURES 9a, 9b et 9c seront décrites.

La cavité métallique 902 est percée en son centre par un trou de forme rectangulaire et dans lequel le circuit imprimé 800 et son antenne 106 sont agencés.

A la différence des FIGURES 9a, 9b et 9c et 10, seules trois faces sur six du circuit imprimé 800 et de son antenne 106 sont recouvertes par une partie métallique de la cavité métallique 902. Ainsi la hauteur h de la cavité métallique 902 est diminuée d'un peu moins de deux tiers de la hauteur h de la cavité métallique 902 illustrée aux FIGURES 9a, 9b et 9c.

Les faces non recouvertes par une partie métallique sont :
- la face avant du circuit-imprimé 800 et de son antenne 106, c'est à dire la face comprenant en son extrémité l'antenne 106 du circuit imprimé 800 et donnant sur la face 902₁ de la cavité métallique 902,
- la face supérieure du circuit imprimé 800 et de son antenne 106, c'est à dire la face du circuit imprimé 800 donnant sur la face 902₅ de la cavité métallique 902, et,
- la face inférieure du circuit imprimé 800 et de son antenne 106, c'est à dire la face du circuit imprimé 800 donnant sur la face 902₆ de la cavité métallique 902.

Le champ magnétique B est parallèle au plan P. La présence de métal sur trois faces sur six du circuit imprimé 800 n'altère pas les performances de rayonnement de l'antenne 106 tant qu'aucune plaque métallique de la cavité métallique 902 ne recouvre le plan formé par l'empilement des couches 106₁ à 106₄ de l'antenne 106 dans la direction A, c'est-à-dire la face à l'extrémité du circuit imprimé 800 crée par l'antenne 106 et donnant sur l'évidement de la face 902₁ de la cavité métallique 902.

Sur les FIGURES 9a, 9b, 9c, 10, 11 et 12, le circuit imprimé 800 est inséré dans une cavité métallique 902. Pour chacun des exemples décrits sur ces FIGURES-ci, avant son insertion dans la cavité métallique 902, le circuit imprimé 800 est encapsulé dans une résine électrique isolante (non illustrée) ou dans une résine non conductrice (non illustrée) puis inséré dans ladite cavité métallique 902, préférentiellement de manière ajustée ou en force. Alternativement, le circuit imprimé 800 encapsulé dans la résine peut être collé dans la cavité métallique 902. Dans ces conditions, une distance minimale d de l'ordre de 0,1 mm peut exister entre le circuit imprimé 800 et les parois intérieures de la cavité métallique 902 assurant ainsi une isolation électrique entre le circuit imprimé 800 et la cavité métallique 902. Bien entendu, en fonction de l'épaisseur de la couche de résine, et le cas échéant de la couche de colle, la distance d peut être supérieure ou égale à 0,1 mm.

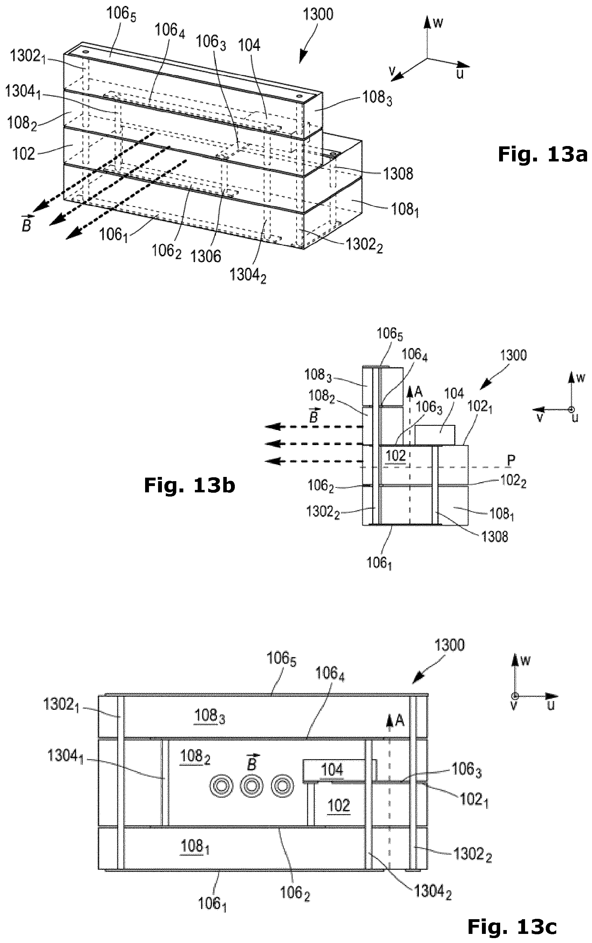

Les FIGURES 13a, 13b et 13c sont des représentations schématiques d'un neuvième exemple du circuit imprimé selon l'invention et de son antenne, respectivement.

Le circuit imprimé 1300 des FIGUES 13a, 13b et 13c est une variante de l'enroulement de l'antenne 106 présenté en FIGURES 8a, 8b et 8c. Ainsi, seules les différences avec les FIGURES 8a, 8b et 8c seront décrites.

La FIGURE 13b est notamment une vue de profil selon un plan (w, v) du circuit imprimé 1300 et de son antenne 106 de la FIGURE 13a et la FIGURE 13c est une vue de face, dans un plan (w,u) du circuit imprimé 1300 et de son antenne 106.

A la différence de l'antenne 106 des FIGURES 8a, 8b et 8c formée par quatre couches de cuivre 106₁, 106₂, 106₃ et 106₄, l'antenne 106 du circuit imprimé 1300 est formée par cinq couches empilées 106₁ à 106s.

Le circuit imprimé 1300 comprend aussi trois supports secondaires 108₁ ,108₂ et 108₃. Notamment, le support secondaire 108₁ est positionné en dessous de la face 102₂ du support principal et le support secondaire 108₂ est positionné au-dessus de la face 102₁ du support principal 102. Le support principal 102 est donc positionné entre les deux supports secondaires 108₁ et 108₂. Le support secondaire 108₃ est positionné au-dessus du support secondaire 108₂.

Les couches 106₁-106₅ de l'antenne 106 sont empilées dans une seule direction A en partant du support secondaire 108₁ du circuit imprimé 800.

Le support principal 102 comprend, sur chacune de ses faces 102₁ et 102₂, une couche empilée 106₃, respectivement 106₂.

La couche empilée 106₁ est agencée sur une face du support secondaire 108₁ opposée à la face 102₂ du support principal 102 de sorte à former une couche extérieure de l'antenne 106 du côté de la face 102₂ du support principal.

La couche empilée 106₅ est agencée sur une face du support secondaire 108₃ opposée au support secondaire 108₂ de sorte à former une couche extérieure de l'antenne 106, du côté de la face 102₁ du support principal 102.

La couche empilée 106₄ est agencée sur une face du support secondaire 108₂ opposée à la face 102₁ du support principal 102.

Sur les FIGURES 13a, 13b, 13c, l'antenne 106 comprend deux vias 1302₁ et 1302₂ traversant le support principal 102 et les supports secondaires 108₁, 108₂ et 108₃ pour relier les couches empilées 106₁ et 106₅ de l'antenne 106 et former un enroulement extérieur de l'antenne 106. De même, l'antenne 106 comprend deux vias 1304₁ et 1304₂.

Le via 1304₁ traverse le support principal 102 et le support secondaire 108₂ et relie les couches 106₂ et 106₄ de l'antenne 106. Le via 1304₂ traverse le support secondaire 108₁, le support principal 102 et le support secondaire 108₂ pour relier les couches 106₁ et 106₄ de l'antenne 106 et former un enroulement intérieur de l'antenne 106.

L'antenne 106 comprend en outre deux autres vias 1306 et 1308.

La via 1306 traverse le support principal 102 et relie ainsi les couches 106₂ et 106₃ de l'antenne 106. Cela permet ainsi de relier le composant électronique 104 à l'enroulement intérieur de l'antenne.

La via 1308 traverse le support secondaire 108₁ et le support principal 102 pour relier les couches 106₁ et 106₃ de l'antenne 106 et ainsi relier le composant électronique 104 à l'enroulement extérieur de l'antenne 106.

Les vias 1302, 1304, 1306, 1308 se présentent sous la forme de trous métalliques réalisés dans le(s) support(s) qu'ils traversent.

La couche 106₄ de l'antenne 106 n'est pas agencée à la même hauteur que la limite supérieure du composant électronique 104. En effet, en FIGURES 13a, 13b et 13c, le composant électronique 104 positionné sur le support principal 102 est en dessous de la limite supérieure du support secondaire 108₂.

Le circuit imprimé 800 comprend aussi, sur sa face 102₁, des connecteurs (non illustrés), sous la forme d'une piste électrique, pour relier l'antenne 106 au composant électronique 104.

Sur les FIGURES 13a à 13c, l'enroulement de l'antenne 106 par l'agencement de ses couches conductrices 106₁, 106₂, 106₃, 106₄, et 106₅ permet un transfert d'énergie optimal dans le plan parallèle au circuit imprimé 1300. En effet, comme représenté en FIGURE 13c, l'enroulement des couches 106₁, 106₂, 106₃, 106₄ et 106s, par les vias 1302, 1304, 1306 et 1308 crée un enroulement de l'antenne 106 perpendiculaire au plan principale P. Ainsi, cela permet au circuit imprimé 1300 de privilégier un rayonnement suivant une direction dans le plan du circuit imprimé 1300, ladite direction étant similaire à celle du champ magnétique B.

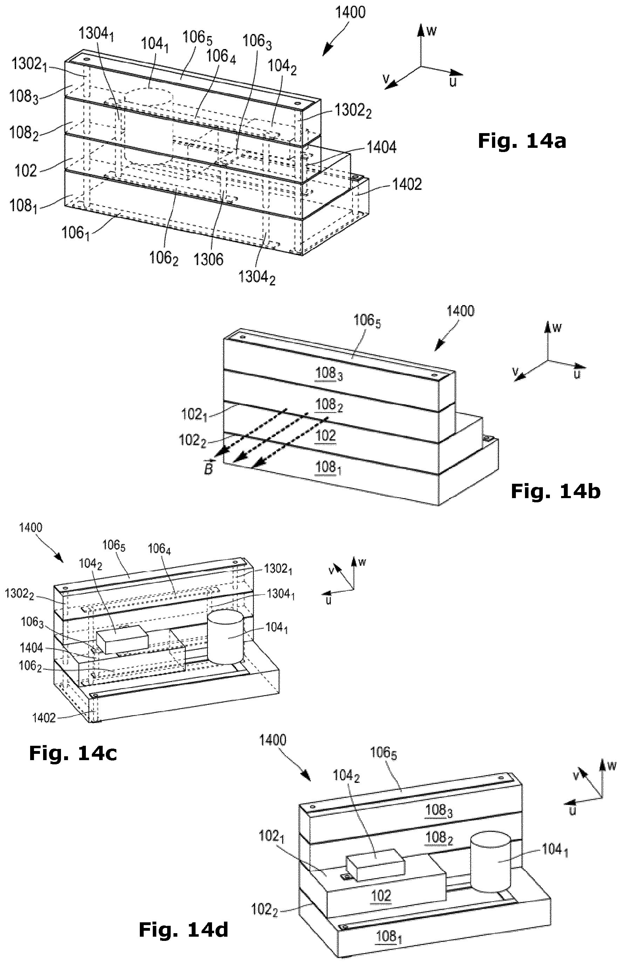

Les FIGURES 14a, 14b et 14c et 14d sont des représentations schématiques d'un dixième exemple du circuit imprimé selon l'invention et de son antenne, respectivement.

Le circuit imprimé 1400 des FIGUES 14a, 14b et 14c et 14d sont une variante d'agencement du circuit imprimé 1300 et de son antenne 106 illustré en FIGURES 13a, 13b et 13c.

La FIGURE 14a est notamment une vue de profil, appelée face avant, du circuit imprimé 1400, ladite FIGURE 14c étant illustrée avec un substrat transparent de manière à distinguer les différents composants du circuit 1400.

La FIGURE 14b est notamment une vue de profil de la face avant du circuit imprimé 1400, sans substrat transparent.

La FIGURE 14c est notamment une vue de profil, appelée face arrière, du circuit imprimé 1400, ladite FIGURE 14c étant illustrée avec un substrat transparent de manière à distinguer les différents composants du circuit 1400.

La FIGURE 14d est notamment une vue de profil, appelée face arrière du circuit imprimé 1400, sans substrat transparent.

Le circuit imprimé 1400 des FIGURES 14a, 14b, 14c et 14d comprend tous les éléments du circuit imprimé 1300. Ainsi, seules les différences avec le circuit imprimé 1300 des FIGURES 13a, 13b et 13c seront décrites.

A la différence du circuit imprimé 1300, le circuit imprimé 1400 comprend deux composants électroniques 104₁ et 104₂.

Le composant 104₁ est positionné sur le support secondaire 108₁.

Le composant 104₁ est une puce, par exemple un RFID standard.

Le composant électronique 104₂ est positionné sur la face 102₁ du support principal 102.

Le composant 104₂ est un composant électronique, par exemple une puce RFID standard, ou un condensateur électrochimique ou un capteur etc.

Le circuit imprimé 1400 comprend en outre des connecteurs (non illustrés), sous forme de piste électrique, pour relier l'antenne 106 aux composants électroniques 104₁ et 104₂. Ainsi, le circuit imprimé 1400 et son antenne 106 permettent l'intégration de composants électroniques 104₁ et 104₂ suivant plusieurs niveaux dans le circuit imprimé 1400, c'est-à-dire qu'ils sont chacun posés et connectés dans des plans différents : un plan pour la face 102₁ pour le composant 104₂ et un autre plan qui lui est parallèle pour la face supérieure du support secondaire 108₁ pour le composant 104₁.

A la différence du circuit imprimé 1300, le circuit imprimé 1400 comprend en outre deux vias 1402 et 1404.

Le via 1404 traverse seulement le support principal 102 et relie les couches 106₃ et 106₂ de l'antenne 106 afin de relier par le biais de la couche 106₂, le composant électronique 104₁ avec le composant électronique 104₂.

Le via 1402 traverse seulement le support secondaire 108₁ et relie les couches 106₁ et 106₂ de l'antenne 106 afin de relier le composant 104₁, à l'enroulement extérieur de l'antenne 106 formé par les couches 106₅ et 106₁.

De manière générale, pour l'ensemble des FIGURES 1 à 14, on comprend que l'antenne 106 est sensible à un champ magnétique B qui se propage selon une direction :
- parallèle au plan principal 102 du circuit imprimé, et
- orthogonal au plan formé par l'empilement des couches de l'antenne 106 selon la direction A.

Bien sûr, l'invention n'est pas limitée aux exemples qui viennent d'être décrits et de nombreux aménagements peuvent être apportés à ces exemples sans sortir du cadre de l'invention selon la revendication 1.

## Revendications

1. Circuit imprimé (100,200,300,400,500) comprenant un support principal (102) s'étendant selon un plan (P), dit principal, un composant électronique (104) disposé sur ledit support principal (102) et une antenne (106), ladite antenne (106) étant :
- formée par une piste conductrice répartie sur aux moins deux couches empilées (106₁,106₂,106₃,106₄) suivant une direction perpendiculaire (A) audit plan principal (P), et
- agencée à une distance (d) non nulle dudit composant électronique (104) dans ledit plan principal (P),
**caractérisé en ce que** l'antenne (106) est une antenne boucle (106,600,700) suivant un plan perpendiculaire audit support principal (102) de sorte que ladite antenne (106) soit sensible à un champ magnétique (B) parallèle audit support principal (102).

2. Circuit imprimé (100,200,300,400,500) selon la revendication précédente, **caractérisé en ce qu'**au moins une face (102₁) du support principal comprend une couche (106₂) de l'antenne.

3. Circuit imprimé (100,200,300) selon l'une quelconque des revendications précédentes, **caractérisé en ce que** chaque face (102₁,102₂) du support principal (102) comprend une couche (106₂,106₁) de l'antenne (106).

4. Circuit imprimé (200,300) selon l'une quelconque des revendications précédentes, **caractérisé en ce que** l'antenne (106) comprend plusieurs couches (106₁,106₂,106₃,106₄) et **en ce que** toutes les couches (106₁,106₂,106₃,106₄) de l'antenne (106) sont empilées d'un même côté par rapport à une face (102₁,102₂) du support principal (106), en particulier une face (102₂) opposée à celle sur laquelle est disposée le composant électronique (104).

5. Circuit imprimé (200,300) selon la revendication précédente, **caractérisé en ce que** toutes les couches (106₁,106₂,106₃,106₄) de l'antenne (106) sont empilées vers le composant électronique (104) à partir de la face (102₂) du support principal opposée à celle comportant ledit composant (104).

6. Circuit imprimé (200,300) selon l'une quelconque des revendications précédentes, **caractérisé en ce que** l'antenne (106) est répartie sur quatre couches empilées (106₁,106₂,106₃,106₄) selon la direction (A) perpendiculaire au plan principal (P).

7. Circuit imprimé (200,300) selon la revendication précédente, **caractérisé en ce que** l'antenne (106) est une antenne boucle formée par deux enroulements (204,206), un enroulement extérieur (204) et un enroulement intérieur (206).

8. Circuit imprimé (100,200,300,400,500) selon l'une quelconque des revendications précédentes, **caractérisé en ce qu'**il comprend au moins un support (108₁,108₂), dit secondaire, comprenant au moins une couche (106₃,106₄) de l'antenne (106) et agencé sur ledit support principal (102).

9. Circuit imprimé (200,300) selon la revendication précédente, **caractérisé en ce qu'**il comprend deux supports secondaires (108₁,108₂) et au moins une couche (106₃) de l'antenne (106) est agencée entre lesdits supports secondaires (108₁,108₂).

10. Circuit imprimé (200,300) selon l'une quelconque des revendications 8 à 9, **caractérisé en ce qu'**au moins une couche (106₄) de l'antenne (106) est agencée sur une face d'un support secondaire (108₂) opposée à l'autre support secondaire (108₁).

11. Circuit imprimé (300) selon l'une quelconque des revendications 9 à 10, **caractérisé en ce que** la piste conductrice comprend au moins un moyen de liaison (304), dit principal, au niveau du support principal (102) et au moins un moyen de liaison (306), dit secondaire, au niveau d'un support secondaire (108₁), lesdits moyens de liaison (304,306) étant prévus pour relier la piste conductrice entre le support principal (102) et ledit support secondaire (108₁).

12. Circuit imprimé (100,200,300,400,500) selon l'une quelconque des revendications précédentes, **caractérisé en ce que** la piste conductrice comprend au moins un via (208,210) reliant deux couches empilées.

13. Circuit imprimé (200,300) selon l'une quelconque des revendications précédentes, **caractérisé en ce qu'**une couche empilée externe (106₄) de l'antenne (106) est à la même hauteur que la limite supérieure du composant électronique (104).

14. Circuit imprimé (100,200,300,400,500) selon l'une quelconque des revendications précédentes, **caractérisé en ce que** le composant électronique (104) est relié à un capteur configuré pour mesurer au moins une donnée relative à l'environnement du circuit imprimé (100,200,300,400,500).

15. Circuit imprimé (1400) selon l'une quelconque des revendications précédentes, ledit composant électronique (104₁) formant un premier composant électronique, **caractérisé en ce qu'**il comprend plusieurs couches (106₁,106₂,106₃,106₄,106₅) de l'antenne (106) agencées pour intégrer au moins un deuxième composant électronique (104₂), le premier composant électronique (104₁) et le deuxième composant électronique (104₂) étant positionnés sur un même niveau dans le circuit imprimé (1400).

16. Circuit imprimé (1400) selon l'une quelconque des revendications 1 à 14, ledit composant électronique (104₁) formant un premier composant électronique, **caractérisé en ce qu'**il comprend plusieurs couches (106₁,106₂,106₃,106₄,106₅) de l'antenne (106) agencées pour intégrer au moins un deuxième composant électronique (104₂), le premier composant électronique (104₁) et le deuxième composant électronique (104₂) étant positionnés sur deux niveaux différents dans le circuit imprimé (1400).

17. Prothèse comprenant un circuit imprimé (100,200,300,400,500) selon l'une quelconque des revendications précédentes.

18. Prothèse selon la revendication précédente, **caractérisée en ce qu'**elle comprend un capteur configuré pour mesurer au moins une donnée relative à ladite prothèse.

## Patentansprüche

1. Gedruckte Schaltung (100, 200, 300, 400, 500), aufweisend einen Hauptträger (102), der sich entlang einer Ebene (P) erstreckt, die als Hauptebene bezeichnet wird, eine elektronische Komponente (104), die auf dem Hauptträger (102) angeordnet ist, und eine Antenne (106), wobei die Antenne (106):
- von einer Leiterbahn gebildet ist, die auf mindestens zwei entlang einer Richtung orthogonal (A) zur Hauptebene (P) gestapelten Schichten (106₁, 106₂, 106₃, 106₄) verteilt ist, und
- in einem Abstand (d) ungleich Null von der elektronischen Komponente (104) in der Hauptebene (P) angeordnet ist,
**dadurch gekennzeichnet, dass** die Antenne (106) eine Schleifenantenne (106, 600, 700) ist, die entlang einer zu dem Hauptträger (102) orthogonalen Ebene verläuft, so dass die Antenne (106) gegenüber einem magnetischen Feld (B) parallel zu dem Hauptträger (102) empfindlich ist.

2. Gedruckte Schaltung (100, 200, 300, 400, 500) nach dem vorhergehenden Anspruch, **dadurch gekennzeichnet, dass** mindestens eine Oberläche (102₁) des Hauptträgers eine Schicht (106₂) der Antenne aufweist.

3. Gedruckte Schaltung (100, 200, 300) nach einem der vorhergehenden Ansprüche, **dadurch gekennzeichnet, dass** jede Oberfläche (102₁, 102₂) des Hauptträgers (102) eine Schicht (106₂, 106₁) der Antenne (106) aufweist.

4. Gedruckte Schaltung (200, 300) nach einem der vorhergehenden Ansprüche, **dadurch gekennzeichnet, dass** die Antenne (106) mehrere Schichten (106₁, 106₂, 106₃, 106₄) aufweist und dass alle Schichten (106₁, 106₂, 106₃, 106₄) der Antenne (106) auf einer gleichen Seite in Bezug auf eine Oberfläche (102₁, 102₂) des Hauptträgers (106) gestapelt sind, insbesondere eine Oberfläche (102₂), die derjenigen gegenüberliegt, auf welcher die elektronische Komponente (104) angeordnet ist.

5. Gedruckte Schaltung (200, 300) nach dem vorhergehenden Anspruch, **dadurch gekennzeichnet, dass** alle Schichten (106₁, 106₂, 106₃, 106₄) der Antenne (106) ausgehend von derjenigen Oberfläche (102₂) des Hauptträgers, welche der die Komponente (104) aufweisenden Oberfläche gegenüberliegt, in Richtung der elektronischen Komponente (104) gestapelt sind.

6. Gedruckte Schaltung (200, 300) nach einem der vorhergehenden Ansprüche, **dadurch gekennzeichnet, dass** die Antenne (106) auf vier Schichten (106₁, 106₂, 106₃, 106₄) verteilt ist, die entlang der zur Hauptebene (P) orthogonalen Richtung (A) gestapelt sind.

7. Gedruckte Schaltung (200, 300) nach dem vorhergehenden Anspruch, **dadurch gekennzeichnet, dass** die Antenne (106) eine Schleifenantenne ist, die durch zwei Wicklungen (204, 206), eine äußere Wicklung (204) und eine innere Wicklung (206), gebildet ist.

8. Gedruckte Schaltung (100, 200, 300, 400, 500) nach einem der vorhergehenden Ansprüche, **dadurch gekennzeichnet, dass** diese mindestens einen Träger (108₁, 108₂) aufweist, der als Sekundärträger bezeichnet wird, der mindestens eine Schicht (106₃, 106₄) der Antenne (106) aufweist und auf dem genannten Hauptträger (102) angeordnet ist.

9. Gedruckte Schaltung (200, 300) nach dem vorhergehenden Anspruch, **dadurch gekennzeichnet, dass** diese zwei Sekundärträger (108₁, 108₂) aufweist und mindestens eine Schicht (106₃) der Antenne (106) zwischen diesen Sekundärträgern (108₁, 108₂) angeordnet ist.

10. Gedruckte Schaltung (200, 300) nach einem der Ansprüche 8 bis 9, **dadurch gekennzeichnet, dass** mindestens eine Schicht (106₄) der Antenne (106) auf einer Fläche eines Sekundärträgers (108₂) angeordnet ist, die dem anderen Sekundärträger (108₁) gegenüberliegt.

11. Gedruckte Schaltung (300) nach einem der Ansprüche 9 bis 10, **dadurch gekennzeichnet, dass** die Leiterbahn mindestens ein Verbindungsmittel (304), das als Hauptverbindungsmittel bezeichnet wird, am Hauptträger (102) und mindestens ein Verbindungsmittel (306), das als Sekundärverbindungsmittel bezeichnet wird, an einem Sekundärträger (108₁) aufweist, wobei die Verbindungsmittel (304, 306) zum Verbinden der Leiterbahn zwischen dem Hauptträger (102) und dem Sekundärträger (108₁) vorgesehen sind.

12. Gedruckte Schaltung (100, 200, 300, 400, 500) nach einem der vorhergehenden Ansprüche, **dadurch gekennzeichnet, dass** die Leiterbahn mindestens eine Durchkontaktierung (208, 210) aufweist, welche zwei gestapelte Schichten verbindet.

13. Gedruckte Schaltung (200, 300) nach einem der vorhergehenden Ansprüche, **dadurch gekennzeichnet, dass** eine äußere gestapelte Schicht (106₄) der Antenne (106) auf der gleichen Höhe wie der obere Rand der elektronischen Komponente (104) liegt.

14. Gedruckte Schaltung (100, 200, 300, 400, 500) nach einem der vorhergehenden Ansprüche, **dadurch gekennzeichnet, dass** die elektronische Komponente (104) mit einem Sensor verbunden ist, der dazu eingerichtet ist, mindestens ein Datenelement bezüglich der Umgebung der gedruckten Schaltung (100, 200, 300, 400, 500) zu messen.

15. Gedruckte Schaltung (1400) nach einem der vorhergehenden Ansprüche, wobei die elektronische Komponente (104₁) eine erste elektronische Komponente bildet, **dadurch gekennzeichnet, dass** diese mehrere Schichten (106₁, 106₂, 106₃, 106₄, 106₅) der Antenne (106) aufweist, die so angeordnet sind, dass sie mindestens eine zweite elektronische Komponente (104₂) integrieren, wobei die erste elektronische Komponente (104₁) und die zweite elektronische Komponente (104₂) auf einem gleichen Niveau in der gedruckten Schaltung (1400) positioniert sind.

16. Gedruckte Schaltung (1400) nach einem der Ansprüche 1 bis 14, wobei die elektronische Komponente (104₁) eine erste elektronische Komponente bildet, **dadurch gekennzeichnet, dass** diese mehrere Schichten (106₁, 106₂, 106₃, 106₄, 106₅) der Antenne (106) aufweist, die so angeordnet sind, dass sie mindestens eine zweite elektronische Komponente (104₂) integrieren, wobei die erste elektronische Komponente (104₁) und die zweite elektronische Komponente (104₂) auf zwei verschiedenen Niveaus in der gedruckten Schaltung (1400) positioniert sind.

17. Prothese, aufweisend eine gedruckte Schaltung (100, 200, 300, 400, 500) nach einem der vorhergehenden Ansprüche.

18. Prothese nach dem vorhergehenden Anspruch, **dadurch gekennzeichnet, dass** diese einen Sensor aufweist, der dazu eingerichtet ist, mindestens ein Datenelement in Bezug auf die Prothese zu messen.

## Claims

1. Printed circuit (100, 200, 300, 400, 500) comprising a primary support (102) extending in a plane (P), called principal plane, an electronic component (104) placed on said primary support (102) and an antenna (106), said antenna (106) being:
- formed by a strip conductor distributed on at least two stacked layers (106₁, 106₂, 106₃, 106₄) in a direction (A) perpendicular to said principal plane (P), and
- arranged at a non-zero distance (d) from said electronic component (104) in said principal plane (P),
**characterized in that** the antenna (106) is a loop antenna (106, 600, 700) in a plane perpendicular to said primary support (102) such that said antenna (106) is sensitive to a magnetic field (B) parallel to said primary support (102).

2. Printed circuit (100, 200, 300, 400, 500) according to the preceding claim, **characterized in that** at least one face (102₁) of the primary support comprises a layer (106₂) of the antenna.

3. Printed circuit (100, 200, 300) according to any one of the preceding claims, **characterized in that** each face (102₁, 102₂) of the primary support (102) comprises a layer (106₂, 106₁) of the antenna (106).

4. Printed circuit (200, 300) according to any one of the preceding claims, **characterized in that** the antenna (106) comprises several layers (106₁, 106₂, 106₃, 106₄) and **in that** all the layers (106₁, 106₂, 106₃, 106₄) of the antenna (106) are stacked on one and the same side with respect to a face (102₁, 102₂) of the primary support (106), in particular a face (102₂) opposite to the one on which the electronic component (104) is placed.

5. Printed circuit (200, 300) according to the preceding claim, **characterized in that** all the layers (106₁, 106₂, 106₃, 106₄) of the antenna (106) are stacked towards the electronic component (104) starting from the face (102₂) of the primary support opposite to the one comprising said component (104).

6. Printed circuit (200, 300) according to any one of the preceding claims, **characterized in that** the antenna (106) is distributed on four stacked layers (106₁, 106₂, 106₃, 106₄) in the direction (A) perpendicular to the principal plane (P).

7. Printed circuit (200, 300) according to the preceding claim, **characterized in that** the antenna (106) is a loop antenna formed by two coils (204, 206), an outer coil (204) and an inner coil (206).

8. Printed circuit (100, 200, 300, 400, 500) according to any one of the preceding claims, **characterized in that** it comprises at least one support (108₁, 108₂), called secondary support, comprising at least one layer (106₃, 106₄) of the antenna (106) and arranged on said primary support (102).

9. Printed circuit (200, 300) according to the preceding claim, **characterized in that** it comprises two secondary supports (108₁, 108₂) and at least one layer (106₃) of the antenna (106) is arranged between said secondary supports (108₁, 108₂).

10. Printed circuit (200, 300) according to any one of claims 8 to 9, **characterized in that** at least one layer (106₄) of the antenna (106) is arranged on a face of a secondary support (108z) opposite to the other secondary support (108₁).

11. Printed circuit (300) according to any one of claims 9 to 10, **characterized in that** the strip conductor comprises at least one connection means (304), called principal connection means, at the level of the primary support (102) and at least one connection means (306), called secondary connection means, at the level of a secondary support (108₁), said connection means (304, 306) being provided to connect the strip conductor between the primary support (102) and the secondary support (108₁).

12. Printed circuit (100, 200, 300, 400, 500) according to any one of the preceding claims, **characterized in that** the strip conductor comprises at least one via (208, 210) connecting two stacked layers.

13. Printed circuit (200, 300) according to any one of the preceding claims, **characterized in that** an outer stacked layer (106₄) of the antenna (106) is at the same height as the upper limit of the electronic component (104).

14. Printed circuit (100, 200, 300, 400, 500) according to any one of the preceding claims, **characterized in that** the electronic component (104) is connected to a sensor configured to measure at least one item of data relating to the environment of the printed circuit (100, 200, 300, 400, 500).

15. Printed circuit (1400) according to any one of the preceding claims, said electronic component (104₁) forming a first electronic component, **characterized in that** it comprises several layers (106₁, 106₂, 106₃, 106₄, 106₅) of the antenna (106) arranged to integrate at least one second electronic component (104₂), the first electronic component (104₁) and the second electronic component (104₂) being positioned on one and the same level in the printed circuit (1400).

16. Printed circuit (1400) according to any one of claims 1 to 14, said electronic component (104₁) forming a first electronic component, **characterized in that** it comprises several layers (106₁, 106₂, 106₃, 106₄, 106₅) of the antenna (106) arranged to integrate at least one second electronic component (104₂), the first electronic component (104₁) and the second electronic component (104₂) being positioned on two different levels in the printed circuit (1400).

17. Prosthesis comprising a printed circuit (100, 200, 300, 400, 500) according to any one of the preceding claims.

18. Prosthesis according to the preceding claim, **characterized in that** it comprises a sensor configured to measure at least one item of data relating to said prosthesis.
